**Europäisches Patentamt**

**European Patent Office**

**Office européen des brevets**

(11) Veröffentlichungsnummer: **0 298 297 B1**

## (12) EUROPÄISCHE PATENTSCHRIFT

(45) Veröffentlichungstag der Patentschrift: **26.02.92**

(21) Anmeldenummer: **88109852.9**

(22) Anmeldetag: **21.06.88**

(51) Int. Cl.⁵: **A61M 35/00**, A61M 37/00

(54) **Transdermales therapeutisches System.**

(30) Priorität: **09.07.87 DE 3722775**

(43) Veröffentlichungstag der Anmeldung:
**11.01.89 Patentblatt 89/02**

(45) Bekanntmachung des Hinweises auf die
Patenterteilung:
**26.02.92 Patentblatt 92/09**

(84) Benannte Vertragsstaaten:
**AT BE CH DE ES FR GB GR IT LI LU NL SE**

(56) Entgegenhaltungen:
**EP-A- 0 117 027**
**EP-A- 0 156 080**
**EP-A- 0 178 601**
**DE-A- 2 314 509**

(73) Patentinhaber: **LTS Lohmann Therapie-**
**Systeme GmbH & Co. KG**
**Irlicherstrasse 55**
**W-5450 Neuwied 12(DE)**

(72) Erfinder: **Becher, Frank**
**Hohenfelder Strasse 5**
**W-5400 Koblenz 1(DE)**

(74) Vertreter: **Neidl-Stippler, Cornelia, Dr.**
**Rauchstrasse 2**
**W-8000 München 80(DE)**

EP 0 298 297 B1

**Beschreibung**

Die Erfindung betrifft ein transdermales therapeutisches System zur Verabreichung von Wirkstoffen an die Haut mit einer der Haut abgewandten Rückschicht, einem Wirkstoffdepot, einer Wirkstoffabgabe-Steuereinrichtung, die die Abgabe des Wirkstoffes durch das System steuert und einer haftklebenden Fixierungseinrichtung für das therapeutische System an der Haut sowie dessen Verwendung.

Gattungsgemäße transdermale Systeme mit mehreren Wirkstoffdepots sind bspw. aus der DE-A-36 29 304 bekannt geworden, wobei ein oder mehrere vereinzelte Wirkstoffdepots ohne Verbindung untereinander in einer Wirkstoffverteilungsmatrix angeordnet sind.

Diese bekannte Anordnung ist insbesondere für feste oder sehr viskose Materialien bevorzugt, wobei sich der hochkonzentrierte Wirkstoff in die Wirkstoffverteilungsmatrix begibt, um von dort aus die - ggf. haftkle-bende - Steuermembran anzufluten.

Aus der EP-A-0117027 (ELAN) ist es bereits bekannt, mehrere getrennte Wirkstoffreservoire in einem transdermalen therapeutischen System vorzusehen, also diskrete Kammern mit einem oder mehreren Wirkstoffen.

Ebenso sind bereits in der DE-A-23 14 509 Mehrkammersysteme in therapeutischen Systemen beschrieben, wobei auch dort keine Verbindung der Kammern miteinander vorhanden ist.

Bei fließfähigen Wirkstoffen bzw. Wirkstoffzubereitungen wird der Wirkstoff häufig in beutelförmigen Ausnehmungen einer Matrix oder in einem aus einer steuernden Membran oder einer Folie gebildeten Beutel aufgenommen, wobei sich bei Erschöpfung des Wirkstoffreservoirs schnell eine Abnahme des Innendrucks und damit der Migrationsgeschwindigkeit des Wirkstoffes ergibt. Ein weiterer Nachteil von Systemen mit großen beutelartigen Einheiten mit einer fluiden Wirkstoffzubereitung besteht darin, daß sie druckempfindlich sind und bei einer Belastung des Systems durch Druck der gesamte Wirkstoff unkontrollierbar aus einem geplatzten Beutel in die Wirkstoffverteilungsmatrix austritt und die Abgabe des Wirkstoffs an die Haut nunmehr nicht mehr gesteuert erfolgt. Dieses ist insbesondere dann unerwünscht, wenn es sich um einen hochwirksamen Wirkstoff handelt, dessen Überdosierung zu Risiken führt.

Ein weiterer Nachteil der bekannten Systeme ist, daß sie nicht teilbar sind, also bspw. für Kinder und Erwachsene bzw. weniger Wirkstoff benötigende Individuen unterschiedliche Größen zu bevorraten sind.

Schließlich stellt sich in einem Pflaster mit einem größeren Flüssigkeitsreservoir bereits durch Schwerkrafteinwirkung eine ungleichmäßige Verteilung der Flüssigkeit ein, die die Gleichmäßigkeit der Wirkstoffabgabe stark beeinträchtigt.

Es ist demzufolge Aufgabe der Erfindung, die obengenannten Nachteile des Standes der Technik zu vermeiden und ein neues transdermales therapeutisches System zu schaffen, das eine sicherere Handhabung insbesondere von flüssigen Wirkstoffen bzw. -zubereitungen ermöglicht.

Die Aufgabe wird erfindungsgemäß durch ein transdermales therapeutisches System gelöst, das dadurch gekennzeichnet ist, daß das Wirkstoffdepot ein Mehrkammersystem ist, in dem diskrete Kammern einen oder mehrere Wirkstoffe aufweisen, die durch Kanäle zumindest partiell miteinander verbunden sind, wobei die Kanäle zwischen den Kammern einen solchen Innendurchmesser aufweisen, daß sie den Durchfluß des Wirstofffluids nur bei Anwendung von Druck erlauben.

Ein Mehrkammersystem ist vorteilhaft, als die Verteilung des Wirkstoffes besser ist und ggf. durch Abschneiden/trennen eines Teilbereichs des transdermalen therapeuti schen Systems dennoch kein Herausfließen oder -fallen der gesamten Wirkstoffzubereitung zu befürchten ist. Ferner können sich fließfähige Wirkstoffzubereitungen unter dem Einfluß von Schwerkraft oder Druck nur begrenzt verschieben, sodaß selbst unter dem Einfluß der Schwerkraft eine ziemlich gleichmäßige Verteilung einer wirkstoffhaltigen Flüssigkeit erzielt werden kann. Falls durch Druckbelastung eine Wirkstoffkammer platzen sollte, bleiben dennoch die restlichen Kammern unbehelligt, so daß die Funktion des Systems noch einigermaßen erhalten bleibt - eine Vorsichtsmaßnahme, die Überdosierungsrisiken bei hochwirksamen flüssigen Wirkstoffen verringert. Dadurch, daß die einzelnen Kammern durch Kanäle zumindest teilweise derart miteinander verbunden sind, daß in Fließen des Kammerinhalts zum Druckausgleich möglich ist, wobei es vorteilhaft ist, daß die Verbindungskanäle zwischen den Kammern eine solchen Innendurchmesser aufweisen, daß sie den Durchfluß des Wirkstofffluids nur bei Anwendung von Druck erlauben. Dies vermeidet das Platzen des Depots und damit das Unbrauchbarwerden des Systems bei Druckbelastung - bspw. bei der Anbringung auf Tieren für veterinärmedizinische Zwecke ist eine derartige Druckverteilungseinrichtung nutzbringend.

Es ist auch möglich, daß das Multikammersystem selbst ein reines Kanalsystem ist.

Dabei können die Kammern jeweils -ggf. unterschiedliche- Wirkstoffabgabe-Steuereinrichtungen aufweisen. Diese Ausführungsform ist insbesondere dann sinnvoll, wenn verschiedene Wirkstoffe in einem System, das diese Wirkstoffe mit unterschiedlicher Abgabegeschwindigkeit an die Haut geben soll, eingesetzt werden.

Die Kammern können in einer oder mehreren, gleichen oder verschiedenen Wirkstoffverteilungsmatrix/ces, die über- und/oder nebeneinander angeordnet sein können, angeordnet sein, also auch auf unterschiedlichen Höhenniveaus des Pflasters, ggf. in unterschiedlichen Verteilungsmatrices.

Ein erfindungsgemäßes transdermales therapeutisches System weist bevorzugt eine ununterbrochene oder unterbrochene Haftklebeschicht als Fixierungseinrichtung auf der Haut auf.

Das transdermale therapeutische System kann ferner eine oder mehrere Haftklebeschichten zwischen Rückschicht und Fixiereinrichtung aufweisen. Dieses ist insbesondere dann notwendig und sinnvoll, wenn die Wirkstoffverteilungsmatrix nicht haftklebend ist und die undurchlässige Rückschicht nur über eine weitere Haftklebeschicht angebracht werden kann.

Das erfindungsgemäße System ist besonders vorteilhaft bei der Anwendung mit einem flüssigen Wirkstoff/en oder bei Wirkstoff in Lösung.

Im Mehrkammersystem können zwischen den Einzelkammern u. ggf. in der Rückschicht und sonstigen Schichten Sollbruchlinien vorgesehen sein. Derartige Sollbruchlinien ermöglichen eine Teilung des Systems, wenn eine geringere Wirkstoffabgabe erwünscht ist. Dies kann bspw. eine aufwendige Lagerhaltung von transdermalen Systemen unterschiedlicher Größen und Dosen vermeiden.

Das Mehrkammersystem kann in einer haftklebenden Wirkstoffverteilungs- und Steuermatrix angeordnet sein.

Das Mehrkammersystem kann dabei aus wirkstoff- bzw. wirkstofflösungsgefüllten Folien mit Steuerwirkung bestehen, die für den Wirkstoff durchlässig, ggf. auch steuernd, sein können und zu einem Mehrkammersystem miteinander verschweißt sind.

Das erfindungsgemäße therapeutische System läßt sich für die Verpackung und Verabreichung von transkutan anwendbaren Wirkstoffen für die Human- und Tiermedizin sowie die Kosmetik einsetzen.

Dabei kann das Depot auch inerte Hilfsstoffe aufweisen. Unter "inert" soll hier verstanden werden, daß Wirkstoff und Hilfsstoff nicht miteinander reagieren; ein "inerter" Hilfsstoff kann auch ein physiologische Wirkungen besitzender Stoff, wie beispielsweise DMSO od. dgl. sein, der beispielsweise die Permeabilität der Haut erhöht. Als derartige Hilfsstoffe bieten sich auch Stützmaterialien an, welche das Wirkstoff-Depot gegenüber Druck- und Zug-Anwendung unempfindlich machen, sowie Trägerstoffe.

Als Wirkstoffe können transdermal anwendbare Wirkstoffe eingesetzt werden. Typische Beispiele dafür sind:

- Nicotin
- Corticosteroide: Hydrocortison, Prednisolon, Beclometason-propionat, Flumetason, Triamcinolon, Triamcinolonacetonid, Fluocinolon,Fluocinolonacetonid, Fluocinolonacetonidacetat, Clobetasol-propionat, usw.
- Analgetische, anti-inflammatorische Mittel, Acetaminophen, Mefenaminsäure, Flufenaminsäure, Diclofenac, Diclofenac-Natrium-Alclofenac, Oxyphenbutazon, Phenylbutazon, Ibuprofen, Flurbiprofen, Salicylsäure, 1-Menthol, Campher, Sulindac-tolmetin-Natrium, Naproxen, Fenbufen, usw.
- Hypnotisch wirksame Sedativa: Phenobarbital, Amobarbital, Cyclobarbital, Triazolam, Nitrazepam, Lorazepam, Haloperidol, usw.
- Tranquilizer: Fluphenazin, Thioridazin, Lorazepam, Flunitrazepam, Chlorpromazin, usw.
- Antihypertensiva: Pindolol, Bufralol, Indenolol, Nifedipin, Lofexidin, Nipradinol, Bucumolol, usw.
- Antihypertensiv wirkende Diuretica: Hydrothiazid, Bendroflumethiazid, Cyclobenthiazid, usw.
- Antibiotica: Penicillin, Tetracyclin, Oxytetracyclin, Fradiomycinsulfat, Erythromycin, Chloramphenicol, usw.
- Anästhetika: Lidocain, Benzocain, Ethylaminobenzoat, usw.
- Antimikrobielle Mittel: Benzalkoniumchlorid, Nitrofurazon, Nystatin, Acetosulfamin, Clotrimazol, usw.
- Antifungus-Mittel: Pentamycin, Amphotericin B, Pyrrolnitrin, Clotrimazol, usw.
- Vitamine: Vitamin A, Ergocalciferol, Colecalciferol, Octotiamin, Riboflavinbutyrat, usw.
- Antiepileptika: Nitrazepam, Meprobamat, Clonazepam, usw.
- Coronar-Vasodilatatoren: Nytroglycenol, Dipyridamol, Erythrittetranitrat, Pentaerythrit-tetranitrat, Propatyl-nitrat, usw.
- Antihistaminika: Diphenylhydramin-hydrochlorid, Chlorpheniramin, Diphenylimidazol, usw.
- Antitussiva: Dextromethorphan (Hydrobromid), Terbutalin(Sulfat), Ephedrin-(Hydrochlorid), Salbutamol (Sulfat), Isoproterenol (Sulfat, Hydrochlorid), usw.
- Sexualhormone: Progesteron, usw.
- Thymoleptika: Doxepin, usw.
- Weitere Arzneimittel: 5-Fluoruracil, Fentanyl, Desmopressin, Domperdon, Scopolamin (Hydrobromid), Peptid, usw.
- selbstverständlich ist diese Aufzählung nicht abschließend.

Vorteilhafterweise kann die Wirkstoffmatrix schichtweise aufgebaut sein, wobei die Schichten gleich oder unterschiedlich sein können. Die Wirkstoffmatrix kann haftklebend sein, beispielsweise ein Gummimaterial, wie styrol/Isopren/Styrol-Blockcopolymerisate, Silicongummi oder auch synthetische Harze, wie Poly(meth)acrylat, Polyurethan, Polyvinylether, Polyester od. dgl. - eine Zusammenfassung geeigneter Matrixmaterialien findet sich z.B. in der DE-OS 35 00 508, auf die vollinhaltlich Bezug genommen wird. Es kann günstig sein wenn die Reservoirmatrix haftklebend ist, da dadurch das Vorsehen einer separat haftklebenden Fixierungseinrichtung im System vermieden werden kannn; die Verwendung einer derartigen haftklebenden Matrix hängt u.a. von der Verträglichkeit des Matrixmaterials mit dem Wirkstoff ab. Haftklebende Matrixmaterialien sind bekannt.

Bevorzugte nicht-haftklebende Matrixmaterialien sind: Polymerisate bestehend aus Poly(meth)-acrylat, Polyvinylpyrrolidon, Ethylcellulose, Hydroxypropylcellulose, Hydroxypropylmethylcellulosephthalat, Polyvinylalkohol bzw. deren Copolymerisate mit Vinyllaurat oder Maleinsäure, Vinylacetat, bzw. dessen Copolymerisat mit Vinyllaurat oder Maleinsäure; Polyvinylether, Butylkautschuk und Polycaprolactam.

Beispielsweise können die Kammern auch zwischen einer rückseitigen Reservoirmatrixschicht und einer hautseitigen Reservoirmatrixschicht eingebracht werden.

Als Rückschicht können an sich bekannte wirkstoffundurchlässige Materialien eingesetzt werden, wie Metallfolien, Kunststoffolien oder auch Laminate derselben, wie sie dem Fachmann auf diesem Gebiet geläufig sind.

Weitere Vorteile und Merkmale ergeben sich aus der nachfolgenden Beschreibung, in der Ausführungsbeispiele der Erfindung anhand der begleitenden Zeichnung näher erläutert sind. Dabei zeigt:

Fig. 1: ein erfindungsgemäßes System im Querschnitt;

Fig. 2: ein weiteres erfindungsgemäßes System im Querschnitt;

Fig. 3: ein weiteres System nach der Erfindung im Querschnitt;

Fig. 4 ein erfindungsgemäßes System in Draufsicht auf das System mit abgenommener Rückschicht

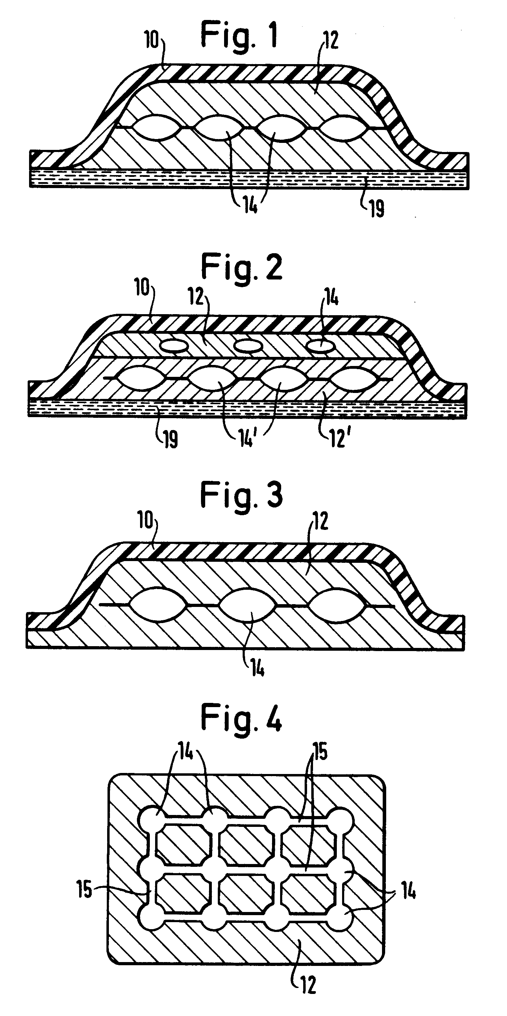

In Fig. 1 ist eine erste bevorzugte Ausführungsform eines erfindungsgemäßen pflasterartigen transdermalen Systems im Querschnitt dargestellt. Das System weist eine wirkstoffundurchlässige Rückschicht 10, wie eine Metallfolie oder eine Kunststoffolie, die Rückschicht kann auch aus einem Folienlaminat aus unterschiedlichen Materialien bestehen, auf. Unter der Rückschicht 10 ist die Wirkstoffverteilungsmatrix 12, die aus einem für den Wirkstoff durchlässigen Material besteht, angeordnet. Für eine derartige Wirkstoffverteilungsmatrix, die bei diesem Ausführungsbeispiel bevorzugt haftklebend ist, eignen sich bspw. selbstvernetzende Acrylatcopolymere; die Matrix ist jedoch auch abhängig vom eingesetzten Wirkstoff. In der Matrix sind Kammern 14 für den Wirkstoff ausgebildet, die mit einem flüssigen Wirkstoff, wie einer Nicotinlösung, gefüllt sind. Zwischen den einzelnen Kammern 14 verlaufen (nicht gezeigte) Kanäle, die nur bei Druck auf das System durchlässig werden und derart das freie Fließen der Nicotinlösung im gesamten Kammersystem 14 im Normalzustand verhindern. Der Wirkstoff, hier das Nicotin, löst sich in der Matrix, wobei die Freisetzungsgeschwindigkeit des transdermalen Systems unter anderem von der Diffusionsgeschwindigkeit des Wirkstoffes in der Wirkstoffmatrix bestimmt wird. Unterhalb der Wirkstoffmatrix, die hier die Kammern 14 völlig umschließt, ist eine Haftkleberschicht 19 aufgetragen, die zur Befestigung des Systems auf der Haut geeignet ist. Diese Haftkleberschicht ist ferner durchlässig für den Wirkstoff und kann ggf. eine steuernde Wirkung auf die Wirkstoffabgabe an die Haut ausüben.

In Fig. 2 ist eine weitere bevorzugte Ausführungsform eines erfindungsgemäßen pflasterartigen transdermalen therapeutischen Systems im Querschnitt darestellt, wobei bei diesem System unterschiedliche Kammern 14,14' in zwei unterschiedlichen Wirkstoffmatrices 12,12' ausgebildet sind, die bspw. unterschiedliche Wirkstoffe bzw. Zubereitungen von Wirkstoffen enthalten können. Auch in dieser Figur ist das Pflaster so geschnitten, daß die Verbindungskanäle zwischen den Kammern nicht gezeigt sind. Die beiden Wirkstoffmatrices können entsprechend den Anforderungen an die jeweilige Wirkstoffabgabegeschwindigkeit ausgewählt sein. Das System weist eine ununterbrochene Klebstoffschicht 19 zum Auflegen auf die Haut bzw. zur Befestigung des Systems auf der Haut, auf, wobei der Klebstoff auch eine gewisse Steuerwirkung ausüben kann.

In Fig. 3 ist eine herstellungsmäßig einfache Ausführungsform der Erfindung im Schnitt dargestellt, bei der die Kammern 14 in einer haftklebenden Wirkstoffmatrix 12 ausgebildet sind, deren Adhäsionseigenschaften auch zur Befestigung des Systems auf der Haut ausreichen (hier verläuft der Schnitt nicht durch Kanäle).

In Fig. 4 ist eine weitere bevorzugte Ausführungsform eines erfindungsgemäßen transdermalen therapeutischen Systems dargestellt, bei der ersichtlich ist, daß die Wirkstoffkammern 14 durch Kanäle 15 miteinander verbunden sind, so daß bspw. bei örtlicher Aufbringung von Druck auf das System die im Kanalsystem/Kammern 14 enthaltene wirkstoffhaltige Flüssigkeit zum Druckausgleich fließen kann.

Bei allen in den Figuren dargestellten Formen können zur weiteren Abgrenzung der Wirkstoffdepots noch beutelartige Folien zwischen Wirkstoffzubereitung und Matrix vorgesehen sein, um die Wirkstoffflüssigkeit besser eingrenzen zu können.

**Patentansprüche**

1. Transdermales therapeutisches System zur Verabreichung von Wirkstoffen an die Haut mit einer der Haut abgewandten Rückschicht (10), einem Wirkstoffdepot (14), einer Wirkstoffabgabe-Steuereinrichtung, die die Abgabe des Wirkstoffes durch das System steuert und einer haftklebenden Fixierungseinrichtung (19) für das therapeutische System an der Haut, dadurch gekennzeichnet, daß das Wirkstoffdepot ein Mehrkammersystem ist, in dem diskrete Kammern (14) einen oder mehrere Wirkstoffe aufweisen, die durch Kanäle (15) zumindest partiell miteinander verbunden sind, wobei die Kanäle (15) zwischen den Kammern (14) einen solchen Innendurchmesser aufweisen, daß sie den Durchfluß des Wirstofffluids nur bei Anwendung von Druck erlauben.

2. Transdermales therapeutisches System gemäß Anspruch 1, dadurch gekennzeichnet, daß die Kammern (14) jeweils - ggf. unterschiedliche - Wirkstoffabgabe-Steuereinrichtungen aufweisen.

3. Transdermales therapeutisches System nach einem der vorangehenden Ansprüche, dadurch gekennzeichnet, daß die Kammern (14) in einer oder mehreren, gleichen oder verschiedenen Wirkstoffverteilungsmatrix/ces (12,12') angeordnet sind.

4. Transdermales therapeutisches System nach einem der vorangehenden Ansprüche, dadurch gekennzeichnet,daß es eine ununterbrochene oder unterbrochene hautseitige Haftklebeschicht (19) als Fixierungseinrichtung auf der Haut aufweist.

5. Transdermales therapeutisches System nach einem der vorangehenden Ansprüche, dadurch gekennzeichnet, daß es ferner eine oder mehrere Haftklebeschichten zwischen Rückschicht (10) und Fixiereinrichtung (19) aufweist.

6. Transdermales therapeutisches System nach einem der vorangehenden Ansprüche, dadurch gekennzeichnet, dadurch gekennzeichnet, daß der Wirkstoff in flüssiger Form oder in Lösung vorliegt.

7. Transdermales therapeutisches System nach einem der vorangehenden Ansprüche, dadurch gekennzeichnet, daß das Mehrkammersystem zwischen den einzelnen Wirkstoffkammern (14) und ggf. in der Rückschicht (10) und sonstigen Schichten Sollbruchlinien (17) aufweist.

8. Transdermales therapeutisches System nach einem der vorangehenden Ansprüche, dadurch gekennzeichnet, daß das Mehrkammersystem ein System von Kanälen (15) ist.

9. Transdermales therapeutisches System nach einem der vorangehenden Ansprüche, dadurch gekennzeichnet, daß die Kammern (14) auf unterschiedlichen Höhenniveaus des Pflasters angeordnet sind, ggf. in unterschiedlichen Verteilungsmatrices.

**Claims**

1. Transdermal therapeutic system for administration of active substances onto the skin with a backing layer facing away from the skin (10), with an active substance depot (14), with an active substance delivery control device which controls the delivery of the active substance by the system, and with an adhesive fixing device (19) for the therapeutic system on the skin, characterised in that the active substance depot is a multi-chamber system in which separate chambers (14) have one or more active substances which are at least partially connected together by channels (15), the internal diameter of the channels (15) between the chambers (14) being such that they permit the active substance fluid to flow through only on application of pressure.

2. Transdermal therapeutic system according to Claim 1, characterised in that the chambers (14) each have - where appropriate different - active substance delivery control devices.

3. Transdermal therapeutic system according to one of the preceding claims, characterised in that the chambers (14) are arranged in one or more, identical or different active substance dispersion matrix/ces (12, 12').

4. Transdermal therapeutic system according to one of the preceding claims, characterised in that it has an uninterrupted or interrupted adhesive layer (19) on the skin side as fixing device to the skin.

5. Transdermal therapeutic system according to one of the preceding claims, characterised in that it furthermore has one or more adhesive layers between backing layer (10) and fixing device (19).

6. Transdermal therapeutic system according to one of the preceding claims, characterised in that characterised in that the active substance is present in liquid form or in solution.

7. Transdermal therapeutic system according to one of the preceding claims, characterised in that the multichamber system has lines (17) of intended breakage between the individual active substance chambers (14) and, where appropriate, in the backing layer (10) and other layers.

8. Transdermal therapeutic system according to one of the preceding claims, characterised in that the multichamber system is a system of channels (15).

9. Transdermal therapeutic system according to one of the preceding claims, characterised in that the chambers (14) are arranged on different vertical levels of the plaster, where appropriate in different dispersion matrices.

**Revendications**

1. Système thérapeutique transdermique permettant d'administer des principes actifs à la peau, comportant une couche arrière opposée à la peau (10), un dépôt de principe actif (14), un dispositif de contrôle de la libération de principe actif, qui contrôle la libération du principe actif à travers le système et un dispositif de fixation autocollant (19) pour fixer le système thérapeutique sur la peau, le système étant caractérisé en ce que le dépôt de principe actif est un système à chambres multiples dans lequel des chambres distinctes (14) présentent un ou plusieurs principes actifs et sont reliées l'une à l'autre au moins partiellement par des canaux (15), lesquels canaux (15) entre les chambres (14) présentent un diamètre intérieur tel qu'ils permettent le passage du fluide de principe actif uniquement lorsque l'on applique une pression.

2. Système thérapeutique transdermique selon la revendication 1, caractérisé en ce que les chambres (14) présentent respectivement des dispositifs de contrôle de libération de principe actif éventuellement différents.

3. Système thérapeutique transdermique selon l'une quelconque des revendications précédentes, caractérisé en ce que les chambres (14) sont agencées dans un ou plusieurs réseaux de distribution de principe actif (12, 12') similaires ou différents.

4. Système thérapeutique transdermique selon l'une quelconque des revendications précédentes, caractérisé en ce qu'il présente une couche autocollante (19) ininterrompue ou interrompue côté peau comme dispositif de fixation sur la peau.

5. Système thérapeutique transdermique selon l'une quelconque des revendications précédentes, caractérisé en ce qu'il présente par ailleurs une ou plusieurs couches autocollantes entre la couche arrière (10) et le dispositif de fixation (19).

6. Système thérapeutique transdermique selon l'une quelconque des revendications précédentes, caractérisé en ce que le principe actif se présente sous forme liquide ou en solution.

7. Système thérapeutique transdermique selon l'une quelconque des revendications précédentes, caractérisé en ce que le système à chambres multiples présente entre les chambres de principe actif individuelles (14) et éventuellement dans la couche arrière (10) et dans les autres couches des lignes de rupture (17).

8. Système thérapeutique transdermique selon l'une quelconque des revendications précédentes, caractérisé en ce que le système à chambres multiples est un système de canaux (15).

9. Système thérapeutique transdermique selon l'une quelconque des revendications précédentes, caractérisé en ce que les chambres (14) sont agencées à des niveaux différents du pansement en hauteur, éventuellement dans différents réseaux de distribution.

Fig. 1

Fig. 2

Fig. 3

Fig. 4